# EUROPEAN PATENT SPECIFICATION

(11) **EP 0 966 263 B1**
(45) Date of publication and mention of the grant of the patent: **02.03.2005**
(21) Application number: 98956320.0
(22) Date of filing: 29.10.1998
(51) Int. Cl.: A61K 7/48, A61K 7/032, A61K 7/027, A61K 7/02, A61K 7/025

(54) **TRANSFER-RESISTANT COLOR COSMETIC COMPOSITION**
FARBÜBERTRAGUNGSBESTÄNDIGE KOSMETISCHE ZUSAMMENSETZUNG
COMPOSITION COSMETIQUE COLOREE RESISTANT AU TRANSFERT

(30) Priority: 31.10.1997 US 962100; 05.12.1997 US 985770
(43) Date of publication of application: 29.12.1999
(73) Proprietor: Color Access, Inc., Melville, New York 11747 (US)
(72) Inventor: KONIK, Richard, A., Sayville, NY 11782 (US); PAINTER, Rachel, J., E. Setauket, NY 11736 (US); STEPNIEWSKI, George, J., Melville, NY 11747 (US); DAVIS, Suzanne, J., Balldwin, NY 11510 (US)
(74) Representative: Pochart, François
(86) International application number: PCT/US1998/022956
(87) International publication number: WO 1999/022710

(56) References cited:
- EP-A- 0 497 144
- WO-A-92/19215
- WO-A-94/12190
- WO-A-94/17775
- WO-A-97/29842
- WO-A-98/42298
- US-A- 5 026 540
- US-A- 5 389 363

## Description

### Related Applications

This application is a continuation-in-part of copending US Serial No. 08/962,100.

### Field of the Invention

The invention relates to cosmetic compositions. More specifically, the invention relates to waterproof and transfer-resistant cosmetic compositions.

### Background of the Invention

There is currently a very strong trend among cosmetics consumers to want products that last the day without the need for refreshing or touching up. It is preferred that a color cosmetic product applies easily, leaving a clear vivid color which remains in place at least through the work day, and preferably into the evening. Given the hectic lifestyles of most consumers, however, providing such a product is not a simple task. Daily physical activity, particularly in the form of daily exercise, which is now so common, is not conducive to makeup retention, with the combination of perspiration and body oils routinely washing away the typical color products with very little effort. In addition, it is also preferred that the product not readily transfer from the place of application. Consumers no longer readily accept a lipstick which leaves its color on a coffee cup, or a foundation which leaves smudges on the collar of a white blouse. Although many currently available products attempt to achieve this desired long-lasting property, there are often other undesirable properties, such as dryness, or difficulty in application, that go along with ability to remain in place on the skin. Thus, there continues to be a need for a color cosmetic which applies smoothly, which is not subject to smearing, flaking, or smudging, and also retains a strong, non-fading color throughout the day. The present invention now provides such a product.

WO-A-9219215 discloses cosmetic waterproofing compositions comprising 5-85% volatile mineral spirit (ie. volatile oil), 0.7-2.1% gellant, 1-50% film-former, and 5-25% of a solvent oil.

WO-A-9729842 discloses solid and semi-solid hydrocarbon gel compositions containing at least one of 5 different types of copolymers, among them styrene-ethylene propylene copolymers.

WO-A-9412190 discloses mineral-oil based health and beauty compositions such as cosmetic, which contain a blend of di- and tri-block copolymers, including styrene-ethylene propylene copolymers.

EP-A-497144 discloses cosmetic compositions containing a particulate styrene-ethylene propylene copolymer, an emollient and a pigment or sunblock agent.

WO-A-9842298 discloses wear-resistant cosmetic compositions comprising a styrene-ethylene-propylene mixed block copolymer, in a volatile solvent, with wax (film-forming agent) and dry powder (pigment).

### Summary of the Invention

The present invention relates to a water-proof or water resistant cosmetic composition for application to the skin comprising a volatile oil solvent, a film-forming agent as below, and a styrene-ethylene-propylene copolymer as gellant. In a preferred embodiment, the composition also contains a pigment. The unpigmented composition can be used for waterproofing non-waterproof color cosmetics, such as a non-waterproof eyeliner. When pigment-containing, the compositions of the invention can be any type of color cosmetic, for example, foundations, blushes, lipsticks or glosses, mascaras for hair and lashes, eyeshadows and eyeliners. The compositions of the invention are waterproof, smudgeproof, non-flaking and when pigmented, transfer-resistant, retaining vibrant color on the skin, with substantially no transfer or fading, for several hours up to a full day.

### Detailed Description of the Invention

The compositions of the invention have a volatile oil base, which provides for a very quick-drying product, which in turn reduces the tendency to smear. Suitable volatile oils for use in the composition include, but are not limited to, both cyclic and linear silicones, such as hexamethylcyclotrisiloxane, octamethylcyclotetrasiloxane, and decamethylcyclopentasiloxane; or straight or branched chain hydrocarbons having from 8-20 carbon atoms, such as decane, dodecane, tridecane,. tetradecane, and C8-20 isoparaffins. Preferred volatile oils are a C8-9 isoparaffin, such as is commercially available from Exxon Corporation, as Isopar E®, or a C₉-C₁₂ aliphatic hydrocarbon, such as is commercially available under the tradename Permethyl®99A, from Permethyl Corp., Frazer, Pennsylvania), or a combination of these. The volatile oil component constitutes from about 1-90%, preferably about 50-85%, by weight of the total composition.

Combined with the volatile oil is at least one film-forming agent, which provides a waterproofing property to the composition, improving the wear of the composition, and also conferring transfer-resistance to the makeup product. The film-former is used in an amount of from about .1-50%, more preferably from about 1-20%. The film-formers is a mixture of PVP/eicosane copolymer and tricontanyl PVP, which produce a smooth, non-tacky film on the skin. Such copolymers are commercially available under the tradename Ganex® from GAF.

In order to incorporate pigment into the volatile oil-film-former combination, it is necessary to add a gellant; in the absence of a gellant, the pigment will simply fall out of suspension. In this type of formula, the most commonly used gellants are clay-based gellants, such as bentone, which when used as the sole gellant, may not always produce a clear gel. Moreover, the use of clay-based gellants like bentone as the sole gellant, depending on the film-forming agent employed, can result in a product which is unstable, allowing leakage of solvents and emollients from the gel matrix. To avoid these problems, a styrene-ethylene-polypropylene copolymer is used as gellant. The use of a styrene-ethylene-polypropylene copolymer as gellant results in a very translucent, non-cloudy, shiny product which permits the true color of the pigment to come through. The pigment readily remains in suspension, and the product. so prepared also retains stability over prolonged periods of time, thereby producing a superior product to one in which a clay-based solvent is used. In addition, even in the absence of a pigment, a desirable viscosity enhancement is achieved by the use of the copolymer.

The copolymer gellants of the invention are particulate diblock copolymers having the formula S-EP, wherein "S" denotes a block comprising styrene monomers and "EP" denotes a block comprising ethylene and propylene monomers. These materials are well known in the art, and are available commercially, for example, from Shell Chemical Company, Oak Brook, Illinois under the tradename "Kraton® G rubber". A particularly preferred material is Kraton® G-1701X. The amount of the gellant used in the formulation is from about 1-15%, more preferably 3-8% by weight of the total composition.

In a preferred embodiment, the composition contains less than 5%, and preferably none, of a non-volatile oil component. The use of a non-volatile oil can cause plasticizing of the film-forming agent, thereby reducing the product's resistance to smudging. The absence of a non-volatile oil thus results in a product with greater wear. With the use of a pliable film-former such as Ganex®, a non-volatile oil is unnecessary to soften it; however, if a harder, or more brittle, film-former is used, a small amount of non-volatile oil may be necessary to achieve the desired consistency of the product.

Additional preferred components of the cosmetic compositions of the invention include one or more pigments. Any cosmetically acceptable pigment, either organic, inorganic, or combinations thereof, can be used in the makeup compositions of the invention. Examples of useful inorganic pigments include iron oxides (yellow, red, brown or black), ferric ammonium ferrocyanide(blue), manganese violet, ultramarine blue, chrome oxide(green), talc, lecithin modified talc, zeolite, kaolin, lecithin modified kaolin, titanium dioxide(white) and mixtures thereof. Other useful pigments are pearlants such as mica, bismuth oxychloride and treated micas, such as titanated micas and lecithin modified micas.

The organic pigments include natural colorants and synthetic monomeric and polymeric colorants. Exemplary are phthalocyanine blue and green pigment, diarylide yellow and orange pigments, and azo-type red and yellow pigments such as toluidine red, litho red, naphthol red and brown pigments. Also useful are lakes, which are pigments formed by the precipitation and absorption of organic dyes on an insoluble base, such as alumina, barium, or calcium hydrates. Particularly preferred lakes are primary FD&C or D&C Lakes and blends thereof.

It will be recognized that when the product is, for example, an eyeliner or other eye product, the pigment should be one the use of which is approved for the eye area. Examples of useful pigments for the eye are metallic oxides, such as titanium or iron oxides, bismuth oxychloride, carmine, chromium oxide or chromium hydroxide greens, ultramarines, ferric ferrocyanide, ferric ammonium ferrocyanide, mica, FD&C blue No. 1, FD&C Red No.40, FD&C yellow No. 5, and FD&C green No. 5. Pigment concentrations will vary depending upon the color of the final product, but generally will be in the range of from about 0.1 to about 30% more preferably from about 1 to about 20%, by weight of the total composition.

The compositions of the invention may also comprise additional, optional components. For example, it may be desirable to add one or more preservatives or antioxidants to the formulation. Appropriate preservatives may include propyl paraben, butyl paraben, mixtures thereof, or isoforms thereof, as well as BHA or BHT.

In particularly preferred embodiments, the compositions of the invention are used as a liquid eyeliner, or as a body paint. In the latter embodiment, the composition can be used to create long-lasting, yet temporary, tattoos or designs on the skin.

The invention is further illustrated by the following non-limiting examples:

### EXAMPLES

### Example I:

A formulation according to the invention is prepared as follows:

| Material | Weight % |
|---|---|
| Phase 1 | |
| C8-9 isoparaffin | 64.85 |

| Phase 2 | |
|---|---|
| styrene-ethylene-propylene copolymer | 5.00 |
| trimethylsiloxysilicate | 5.00 |
| PVP/eicosene copolymer | 5.00 |
| tricontanyl PVP | 5.00 |
| polyethylene | 5.00 |
| isododecane/quaternium-18 hectorite | 0.10 |
| BHT | 0.10 |

| Phase 3 | |
|---|---|
| iron oxides/methicone | 10.00 |

The Phase 2 components are dissolved in Phase 1 component at about 90°C, and mixed to homogeneity. Phase 3 components are then added to the mixture until homogeneously dispersed.

The product so prepared is stable, waterproof, and highly resistant to smudging.

## Claims

1. A waterproof or water resistant cosmetic composition comprising a styrene-ethylene-propylene copolymer as gellant, a film forming agent, and a volatile oil, in which the film-forming agent comprises a mixture of PVP/eicosene copolymer and a tricontanyl PVP copolymer.

2. The composition of claim 1 which also comprises a pigment.

3. The composition of claim 1 or 2 in which the volatile oil is selected from the group consisting of cyclic and linear silicones, straight or branched chain hydrocarbons having from 8-20 carbon atoms, and C8-20 isoparaffins.

4. The composition of claim 3 in which the volatile oil is a C8-9 isoparaffin.

5. The composition of any one of claims 1 to 4 in which the styrene-ethylene-propylene copolymer is present in an amount of from about 1 to about 15%.

6. The composition of any one of claims 1 to 5 in which the film-forming agent is present in an amount of from about 0.1 to about 50%.

7. The composition of any one of claims 1 to 6 in which the volatile oil is present in an amount of from about 1 to about 90%.

8. The composition of any one of claims 1 to 7 comprising a styrene-ethylene-propylene copolymer in an amount of from about 1 to about 15%, a PVP copolymer in an amount of from about 0.1 to about 50%, a volatile oil in an amount of from about 1 to about 90%, and a pigment in an amount of from about 1 to about 30%.

9. The composition of claim 8 comprising a styrene-ethylene-propylene copolymer in an amount of from about 5 to about 10%, a PVP copolymer in an amount of from about 1 to about 20%, and a volatile oil in an amount of from about 50 to about 85%, and a metallic oxide pigment in an amount of from about 1 to about 30%.

10. The composition of claim 8 or 9 in which the volatile oil is a C8-9 isoparaffin, a C9-C12 aliphatic hydrocarbon, or a combination thereof.

11. The composition of any one of claims 8 to 10 which comprises less than 5% of a non-volatile oil.

12. The composition of claim 11 which comprises substantially no non-volatile oil.

13. The composition of claim 1 which comprises a styrene-ethylene-propylene copolymer in an amount of from about 5 to 10%, a combination of a PVP/eicosene copolymer and a tricontanyl PVP copolymer in an amount of from about 1 to 20%, a C8-9 isoparaffin, a C9-C12 aliphatic hydrocarbon, or a combination thereof, in an amount of from about 50 to 85%.

14. ,- The composition of claim 13 which also comprises a pigment in an amount of from about 1 to about 30%.

15. The composition of claim 13 or 14 which comprises a styrene-ethylene-propylene copolymer in an amount of from about 5 to about 10%. a combination of a PVP/eicosene copolymer and a tricontanyl PVP copolymer in an amount of from about 1 to about 20%, a C8-9 isoparaffin, a C9-C12 aliphatic hydrocarbon, or a combination thereof, in an amount of from about 50 to about 85%, and a metallic oxide pigment in an amount of from about 1 to about 30%.

16. The composition of any one of claims 13 to 15 which also comprises at least one other film-forming agent, in an amount of from about 1-10%.

## Patentansprüche

1. Eine wasserfeste oder wasserresistente kosmetische Zusammensetzung, die ein Styrol-Ethylen-Propylen-Copolymer als Schaumfestiger, ein filmbildendes Agens, ein flüchtiges Öl, wobei das filmbildende Agens eine Mixtur aus PVP/Eicosen-Copolymer und einem Tricontanyl PVP-Copolymer umfasst, umfasst.

2. Die Zusammensetzung gemäß Anspruch 1, die auch ein Pigment umfasst.

3. Die Zusammensetzung gemäß Anspruch 1 oder 2, in der das flüchtige Öl aus der Gruppe ausgewählt wird, die aus zyklischen und linearen Silikonen, geraden oder verzweigten Kohlenwasserstoffketten mit 8-20 Kohlenstoffatomen und C8-20 Isoparaffinen besteht.

4. Die Zusammensetzung gemäß Anspruch 3, in der das flüchtige Öl ein C8-9 Isoparaffin ist.

5. Die Zusammensetzung gemäß einem der Ansprüche 1 bis 4, in der das Styrol-Ethylen-Propylen-Copolymer in einer Menge von etwa 1 bis etwa 15% vorhanden ist.

6. Die Zusammensetzung gemäß einem der Ansprüche 1 bis 5, in der das filmbildende Agens in einer Menge von etwa 0,1 bis etwa 50% vorhanden ist.

7. Die Zusammensetzung gemäß einem der Ansprüche 1 bis 6, in der das flüchtige Öl in einer Menge von etwa 1 bis etwa 90% vorhanden ist.

8. Die Zusammensetzung gemäß einem der Ansprüche 1 bis 7, die ein Styrol-Ethylen-Propylen-Copolymer in einer Menge von etwa 1 bis etwa 15%, ein PVP-Copolymer in einer Menge von etwa 0,1 bis etwa 50%, ein flüchtiges Öl in einer Menge von etwa 1 bis etwa 90% und ein Pigment in einer Menge von etwa 1 bis etwa 30% umfasst.

9. Die Zusammensetzung gemäß Anspruch 8, die ein Styrol-Ethylen-Propylen-Copolymer in einer Menge von etwa 5 bis etwa 10%, ein PVP-Copolymer in einer Menge von etwa 1 bis etwa 20%, und ein flüchtiges Öl in einer Menge von etwa 50 bis etwa 85% und ein metallisches Oxidpigment in einer Menge von etwa 1 bis etwa 30% umfasst.

10. Die Zusammensetzung gemäß Anspruch 8 oder 9, in der das flüchtige Öl ein C8-9 Isoparaffin, ein C9-C12 aliphatischer Kohlenwasserstoff oder eine Kombination davon ist.

11. Die Zusammensetzung gemäß einem der Ansprüche 8 bis 10, die weniger als 5% eines nichtflüchtigen Öles umfasst.

12. Die Zusammensetzung gemäß Anspruch 11, die im Wesentlichen ein nichtflüchtiges Öl umfasst.

13. Die Zusammensetzung gemäß Anspruch 1, die ein Styrol-Ethylen-Propylen-Copolymer in einer Menge von etwa 5 bis 10%, eine Kombination eines PVP/Eicosen-Copolymers und eines Tricontanyl PVP-Copolymers in einer Menge von etwa 1 bis 20%, ein C8-9 Isoparaffin, einen C9-C12 aliphatischen Kohlenwasserstoff oder eine Kombination davon in einer Menge von etwa 50 bis 85% umfasst.

14. Die Zusammensetzung gemäß Anspruch 13, die auch ein Pigment in einer Menge von etwa 1 bis etwa 30% umfasst.

15. Die Zusammensetzung gemäß Anspruch 13 oder 14, die ein Styrol-Ethylen-Propylen-Copolymer in einer Menge von etwa 5 bis 10%, eine Kombination eines PVP/Eicosen-Copolymers und eines Tricontanyl PVP-Copolymers in einer Menge von etwa 1 bis 20%, ein C8-9 Isoparaffin, einen C9-C12 aliphatischen Kohlenwasserstoff oder eine Kombination davon in einer Menge von etwa 50 bis 85%, und ein metallisches Oxidpigment in einer Menge von etwa 1 bis 30% umfasst.

16. Die Zusammensetzung gemäß einem der Ansprüche 13 bis 15, die auch mindestens ein anderes filmbildendes Agens in einer Menge von etwa 1-10% umfasst.

## Revendications

1. Composition cosmétique hydrofuge ou résistant à l'eau comprenant un copolymère styrène-éthylène-propylène en tant qu'agent gélifiant, un agent filmogène et une huile volatile, dans laquelle l'agent filmogène comprend un mélange de copolymère PVP/eicosène et d'un copolymère tricontanyl PVP.

2. Composition selon la revendication 1, qui comprend aussi un pigment.

3. Composition selon la revendication 1 ou 2, dans laquelle l'huile volatile est choisie dans le groupe consistant en silicones cycliques et linéaires, hydrocarbures à chaîne droite ou ramifiée ayant de 8 à 20 atomes de carbone et isoparaffines en C₈₋₉.

4. Composition selon la revendication 3, dans laquelle l'huile volatile est une isoparaffine en C₈₋₉.

5. Composition selon l'une quelconque des revendications 1 à 4, dans laquelle le copolymère styrène-éthylène-propylène est présent en une quantité d'environ 1 à environ 15%.

6. Composition selon l'une quelconque des revendications 1 à 5, dans laquelle l'agent filmogène est présent en une quantité d'environ 0,1 à environ 50%.

7. Composition selon l'une quelconque des revendications 1 à 6, dans laquelle l'huile volatile est présente en une quantité d'environ 1 à environ 90%.

8. Composition selon l'une quelconque des revendications 1 à 7, comprenant un copolymère styrène-éthylène-propylène en une quantité d'environ 1 à environ 15%, un copolymère PVP en une quantité d'environ 0,1 à environ 50%, une huile volatile en une quantité d'environ 1 à environ 90% et un pigment en une quantité d'environ 1 à environ 30%.

9. Composition selon la revendication 8, comprenant un copolymère styrène-éthylène-propylène en une quantité d'environ 5 à environ 10%, un copolymère PVP en une quantité d'environ 1 à environ 20% et une huile volatile en une quantité d'environ 50 à environ 85%, et un pigment de type oxyde métallique en une quantité d'environ 1 à environ 30%.

10. Composition selon la revendication 8 ou 9, dans laquelle l'huile volatile est une isoparaffine en C₈₋₉, un hydrocarbure aliphatique en C₉₋₁₂ ou une combinaison de ceux-ci.

11. Composition selon l'une quelconque des revendications 8 à 10, qui comprend moins de 5% d'une huile non volatile.

12. Composition selon la revendication 11, qui comprend substantiellement aucune huile non volatile.

13. Composition selon la revendication 1, qui comprend un copolymère styrène-éthylène-propylène en une quantité d'environ 5 à 10%, une combinaison d'un copolymère PVP/eicosène et d'un copolymère tricontanyl PVP en une quantité d'environ 1 à 20%, une isoparaffine en C₈₋₉, un hydrocarbure aliphatique en C₉₋₁₂ ou une combinaison de ceux-ci, en une quantité d'environ 50 à environ 85%.

14. Composition selon la revendication 13, qui comprend aussi un pigment en une quantité d'environ 1 à environ 30%.

15. Composition selon la revendication 13 ou 14, qui comprend un copolymère styrène-éthylène-propylène en une quantité d'environ 5 à environ 10%, une combinaison d'un copolymère PVP/eicosène et d'un copolymère tricontanyl PVP en une quantité d'environ 1 à environ 20%, une isoparaffine en C₈₋₉, un hydrocarbure aliphatique en C₉₋₁₂ ou une combinaison de ceux-ci, en une quantité d'environ 50 à environ 85%, et un pigment de type oxyde métallique en une quantité d'environ 1 à environ 30%.

16. Composition selon l'une quelconque des revendications 13 à 15, qui comprend aussi au moins un autre agent filmogène, en une quantité d'environ 1 à 10%.
